# EUROPEAN PATENT SPECIFICATION

(11) **EP 1 307 185 B1**
(45) Date of publication and mention of the grant of the patent: **13.04.2005**
(21) Application number: 01958375.6
(22) Date of filing: 10.08.2001
(51) Int. Cl.: A61K 31/18, A61K 31/381, A61K 31/382, A61P 3/10, A61P 27/06, A61K 31/425

(54) **Use of carboanhydrase inhibitors for the prevention of diabetic retinopathy in diabetics**
Verwendung von Carboanhydrase-Inhibitoren zur Prävention von diabetischer Retinopathie bei Diabetikern
Utilisation d'inhibiteurs d'anhydrase carbonique pour la prévention de la rétinopathie diabétique chez les diabétiques

(30) Priority: 11.08.2000 US 224358 P
(43) Date of publication of application: 07.05.2003
(73) Proprietor: Stefansson, Einar, 110 Reykjavik (IS)
(72) Inventor: Stefansson, Einar, 110 Reykjavik (IS)
(74) Representative: Hedley, Nicholas James Matthew
(86) International application number: PCT/IS2001/000015
(87) International publication number: WO 2002/013800

(56) References cited:
- EP-A- 0 079 269
- WO-A-99/44603
- US-A- 5 948 801
- US-B1- 6 242 442
- DATABASE BIOSIS [Online] BIOSCIENCES INFORMATION SERVICE, PHILADELPHIA, PA, US; PREV199799762187, GROVER SABDEEP ET AL: "Efficacy of dorzolamide hydrochkloride in the management of chronic cystoid macular edema in patients with retinitis pigmentosa" XP002902284
- KOHNER ET AL: 'Role of Blood Flow and Impaired Autoregulation in the Pathogenesis of Diabetic Retinopathy' DIABETES vol. 44, 01 June 1995, pages 603 - 607, XP009031369
- KOHNER ET AL: 'The Retinal Blood Flow in Diabetes' DIABETE AND METABOLISME vol. 19, 1993, pages 401 - 404, XP009031370

## Description

### BACKGROUND OF THE INVENTION

### Field of the Invention:

The invention relates to the preparation of a medicament for the prevention of diabetic retinopathy.

### Background of the Prior Art:

Diabetic retinopathy is a major cause of blindness in the Western world; see, for example, Klein et al., *Ophthalmology* **91**, 1-9 (1984); Klein et al., in *Retina and Vitreous,* ed. Grand et al., American Academy of Ophthalmology, San Francisco, CA, Chapter 5, 70 (1997). Retinal blood vessels are adversely affected and capillary occlusions are a prominent feature. Retinal edema, neovascularization and hemorrhages lead to reduced vision; see Stefánsson et al., in *Diabetic Renal-Retinal Syndrome Prevention and Management,* ed. Friedman et al., Grune and Stratton, New York, 117-150 (1982); Stefánsson et al., *Ophthalmic Surgery* **14,** 209-226 (1983).

Apart from the general treatment of diabetes mellitus and blood pressure, the treatment of diabetic retinopathy is at the present time limited to retinal laser photocoagulation and vitreous surgery: Kristinsson, *Diabetic Retinopathy Screening and Prevention of Blindness* (Ph.D. thesis), University of Iceland, Reykjavík, Iceland, Chapter 1, 15-28 (1996). Laser photocoagulation and vitreous surgery are used in advanced sight-threatening retinopathy. No specific treatment exists for treatment or prevention of mild or moderate retinopathy or prevention of retinopathy in diabetics with no retinopathy. Thus, there is serious need in this art for alternate methods for the treatment of diabetic retinopathy, and especially for a less invasive method of treating diabetic retinopathy as well as other retinopathies.

The improvement of retinal oxygen tension appears to be a key factor in the treatment benefit from retinal photocoagulation and vitrectomy: Stefánsson, *Graefe's Archiv. Clin. Exp. Ophthalmol.,* **228**, 120:123 (1990); Novack et al., *Exp. Eye. Res.* **50**: 289-296 (1990); Stefánsson et al., *Trans Am. Ophthal. Soc.* **79**, 307-334 (1981); Stefánsson et al., *Am. J. Ophthalmology* **101,** 657-664 (1986); Stefánsson et al., *Am. J. Ophthalmology,* **113,** 36-38 (1992).

Carbonic anhydrase inhibitors (CAIs) are known to be effective in the treatment of glaucoma, an ocular disorder associated with elevated ocular pressures. If untreated, glaucoma may lead to blindness. Ocular hypertension, that is, elevated intraocular pressure without optic nerve head damage or characteristic glaucomatous visual field defects, may represent the earliest phase of glaucoma.

It has recently been suggested by the present inventor and his co-workers that the carbonic anhydrase inhibitors dorzolamide and acetazolamide may affect the glaucomatous optic nerve in the pig through two distinct mechanisms. One of these is the traditional intraocular pressure lowering effect, and the other is the direct effect on optic nerve oxygen tension (ONPo₂). Acetazolamide and dorzolamide increased ONPo₂ in the pig. See Stefánsson et aL, *Investigative Ophthalmology and Visual Science (IOVS),* October 1999, Vol. 40, No. 11, 2756-2761. The vasculature of the pig optic nerve head has features, which are in common with the human, as well as features which are different.

W099 44603 describes a method of treating macular disorders to improve visual function by means of a carbonic anhydrase inhibitor in combination with an ocular hypotensive agent. The macular disorder may be associated with diabetic retinopathy.

US 5984801 describes a method for treating retinal edema using a topical application of brizolamine, which is a carbonic anhydrase inhibitor.

*RETINA (Journal of Retinal and Vitreous Diseases), 17(3), 222-231 (1997), Grover, S et al* describes a study of the topical application of one carbonic anhydrase inhibitor (dorzolamide hydrochloride) with the oral administration of another carbonic anhydrase inhibitor (acetazolamide) in the management of cystoid macular edema in patients with retinitis pigmentosa.

### OBJECTS AND SUMMARY OF THE INVENTION

The present invention provides use of a carbonic anhydrase inhibitor in the preparation of a medicament for the prevention of diabetic retinopathy in a diabetic not suffering from diabetic retinopathy.

### DETAILED DESCRIPTION OF THE INVENTION AND PREFERRED EMBODIMENTS

The term "prevention" as used herein means the act of keeping from happening, either temporarily or permanently.

The present invention applies to any type of diabetes mellitus including type 1 (insulin dependent diabetes mellitus) and type 2 (non-insulin dependent diabetes mellitus).

The term "carbonic anhydrase inhibitor" as used herein means an agent which blocks or impedes the carbonic anhydrase pathway by inhibiting the enzyme, carbonic anhydrase. Carbonic anhydrase inhibitors which can be directed only or principally to the desired ocular target tissue are particularly useful in the present invention; however, any carbonic anhydrase inhibitor falls within the purview of the present invention.

Carbonic anhydrase inhibitors (CAIs) as a class are well-known; these compounds have found widespread acceptance in the treatment of elevated intraocular pressure, especially glaucoma. Some of these compounds have also been used as diuretics, for example, in the treatment of congestive heart failure, or in the treatment of allergies. See, for example, the following patents relating to compounds of this type: U.K. Patent Specification No. 769,757; Clapp et al. U.S. Patent No. 2,554,816, Young et al. U.S. Patent No. 2,783,241; Schultz U.S. Patent No. 2,835,702; Korman U.S. Patent No. 2,868,800; Yale et al. U.S. Patent No. 3,040,042; Sircar et al. U.S. Patent No. 4,092,325; Woltersdorf, Jr. et al. U.S. Patent No. 4,386,098; Woltersdorf, Jr. et al. U.S. Patent No. 4,416,890; Woltersdorf, Jr. U.S. Patent No. 4,426,388; Shepard U.S. Patent No. 4,542,152; EP 0182691; Shepard et al. U.S. Patent No. 4,668,697; Baldwin et al. U.S. Patent No. 4,677,115; EP 0228237; Baldwin et al U.S. Patent No. 4,797,413; and Marin U.S. Patent No. 4,619,939.

Generally speaking, carbonic anhydrase inhibitors have a sulfonamide structure which is attached to a ring system; typically, CAIs are heterocyclic or aryl sulfonamides. At the present time, preferred CAIs for use in the present invention include dorzolamide, acetazolamide, brinzolamide, methazolamide, ethoxzolamide (ethoxyzolamide), butazolamide, dichlorphenamide and flumethiazide. The chemical names for these preferred agents are as follows:
- dorzolamide:: (4S-trans)-4-(ethylamino)-5,6-dihydro-6-methyl-4H-thieno[2,3-b]thiopyran-2-sulfonamide dioxide
- acetazolamide:: N-[5-(aminosulfonyl)-1,3,4-thiadiazol-2-yl]acetamide
- brinzolamide:: (R)-(+)-4-ethylamino-2-(3-methoxypropyl)-3,4-dihydro-2H-thieno[3,2-e]-1,2-thiazine-6-sulfonamide-1,1-dioxide
- methazolamide:: N-[5-(aminosulfonyl)-3-methyl-1,3,4 thiadiazol-2(3H)-ylidene]acetamide
- ethoxyzolamide:: 6-ethoxy-2-benzothiazolesulfonamide
- butazolamide:: N-[5-(aminosulfonyl)-1,3,4-thiadiazol-2-yl)butanamide
- dichlorphenamide:: 4,5-dichloro-1,3-benzenedisulfonamide
- flumethiazide:: 6-(trifluoromethyl)-2H-1,2,4-benzothiadiazine-7-sulfonamide 1,1-dioxide
See also *The Merck Index,* 12^{th} edition, ed Susan Budavari et al. Merck & Co., Inc., Whitehouse Station, New Jersey, 1996, pp. 10, 250, 520, 579, 641, 701, 1020; and *Physicians' Desk Reference,* 54^{th} edition, Medical Economics Company, Inc., Montvale, New Jersey, 2000, pp. 496-487, 1767-1769,1778-1779, 1897-1898. Other suitable CAIs for use in the present invention will be apparent to those of ordinary skill in the art.

Improvement in the oxygen tension in the retina and optic nerve results from retinal photocoagulation and vitrectomy, the current methods for treating diabetic retinopathy. It has now been found in studies of select carbonic anhydrase inhibitors in the pig that CAIs have improved the oxygen tension in the retina and optic nerve. The present inventor has seen an analogy in these studies, leading to his proposal to use CAIs instead of the current treatment for diabetic retinopathy. CAI treatment is less invasive and less destructive than laser photocoagulation and vitrectomy and, in addition, is applied at an earlier stage in a preventative mode. The present inventor thus proposes using dorzolamide and other carbonic anhydrase inhibitors such as acetazolamide, methazolamide, brinzolamide, ethoxyzolamide, benzolamide and the like in the prevention of diabetic retinopathy in a patient not suffering diabetic retinopathy.

It is apparent from the foregoing that the present invention is of particular interest when applied to humans. However, the invention is applicable to other mammals as well, for example, dogs and cats.

Some of the carbonic anhydrase inhibitors are particularly active systemically and thus can be formulated for administration by a systemic route (such as oral or parenteral) in accord with the present invention. Other CAIs are particularly active locally and are suitable for topical/ophthalmic formulation and administration to the eye or eyes in accord with this invention. Yet other CAIs can be administered by both systemic and local routes in the practice of the present invention. Generally, even the topically active CAIs can be absorbed systemically following ophthalmic administration. Ophthalmic administration is nevertheless a particularly preferred type of administration in accord with the present invention, and particularly preferred CAIs for administration by this route include dorzolamide and brinzolamide. In the case of parenteral-administration, dorzolamide and acetazolamide are particularly preferred CAIs. In the case of oral administration, preferred CAIs are methazolamide and acetazolamide. Ophthalmic or oral administration is preferred, especially when self-dosing over an extended period is desired. Selection of a particular CAI will depend upon various factors, for example, the nature and severity of the retinopathy, the species to which the drug is administered, the size, condition and age of the patient, and the route of administration desired. The choice of the particular formulation and dosage level similarly will depend upon these various factors and upon the identity of the selected CAI. At the present time, preferred CAIs for use in the treatment of retinopathy in accord with the present invention include dorzolamide, acetazolamide, brinzolamide, methazolamide, ethoxzolamide, butazolamide, dichlorphenamide, flumethiazide and related compounds.

The carbonic anhydrase inhibitors or pharmaceutically acceptable salts thereof can be conveniently administered in the form of a pharmaceutical composition comprising the selected CAI or its salt (e.g., an acid addition salt such as the hydrochloride) and a pharmaceutically acceptable carrier therefore. Suitable carriers vary with the desired form of the pharmaceutical composition and may include diluents or excipients such as fillers, binders, wetting agents, disintegrators, surface-active agents, lubricants and the like.

The selected CAI or its salt, in an amount effective to prevent or treat retinopathy, may be formulated together with the carrier into any desired unit dosage form, the dosage form of choice depending upon the ultimate use of the selected CAI and the route or routes of administration for which it is best suited. Typical unit dosage forms include tablets, pills, powders, solutions, suspensions, emulsions, gels, ointments, granules, capsules and suppositories. Solutions and suspensions formulated as eye drops are especially preferred.

In the preparation of tablets, carriers which are widely used in this field can be employed, e.g. excipients such as lactose, sucrose, sodium chloride, glucose solution, urea, starch, calcium carbonate, kaolin, crystalline cellulose, cyclodextrins and silicic acid; binding agents such as water, ethanol, propanol, simple syrup, glucose, starch solution, gelatin solution, carboxymethyl cellulose, shellac, methyl cellulose, calcium phosphate and polyvinylpyrrolidone; disintegrators such as dried starch, sodium alginate, agar-agar powder, laminalia powder, sodium bicarbonate, calcium carbonate, Tweens, sodium lauryl sulfate, stearic acid monoglyceride, starch and lactose; disintegration inhibitors such as sucrose, stearin, coconut butter and hydrogenated oil; absorption accelerators such as quaternary ammonium bases and sodium lauryl sulfate; wetting agents such as glycerin and starch; adsorbing agents such as starch, lactose, kaolin, bentonite and colloidal silicic acid; and lubricants such as purified talc, stearic acid salts, boric acid powder, Macrogol and solid polyethylene glycol.

In the preparation of pills, carriers which are known and widely used in this field can also be used, for example, excipients such as glucose, lactose, starch, coconut butter, hydrogenated vegetable oils, kaolin and talc; binders such as powdered gum arabic, powdered tragacanth, gelatin and ethanol; and disintegrators such as laminaria and agar-agar. In the case of tablets, they can be further coated with the usual coating materials to make sugar-coated tablets, gelatin film-coated tablets, tablets coated with enteric coatings, tablets coated with films or double-layered tablets and multi-layered tablets.

In order to form suppositories, carriers which are known and widely used in this field can also be used, for example, polyethylene glycols, coconut butter, higher alcohols, esters of higher alcohols, gelatin and semi-synthesized glycerides.

Furthermore, the usual dissolving agents, buffers, analgesic agents and preservatives can be added, as well as coloring materials, perfumes, seasoning agents, sweetening agents and other medicines, to the pharmaceutical compositions, if necessary or if desired.

The amount of a CAI or its salt to be present in the pharmaceutical composition can be suitably be selected from a wide range, but usually 1 to 70% by weight of the total composition is preferable when the composition is a solid dosage form.

As to the route of administration, same will vary with the particular composition used. For example, tablets, pills, solutions, suspensions, emulsions, granules and capsules can be administered orally; suppositories can be administered rectally.

The dosage of the carbonic anhydrase inhibitor or its salt and frequency of administration is selected according to the usage, purpose and conditions of symptoms, as well as the size and species of the recipient.

The CAIs and their salts which are topically active can be conveniently administered in accord with the invention by formulating the selected compound or salt, in an amount effective to prevent or treat retinopathy, together with a non-toxic ophthalmically acceptable carrier therefor. Suitable carriers will be apparent to those skilled in the art of ophthalmic formulations. Obviously, the choice of suitable carriers will depend on the exact nature of the particular dosage form desired, e.g. whether the CAI or its salt is to be formulated into an ophthalmic solution or suspension (typically for use as eye drops), an ophthalmic ointment or cream or an ophthalmic gel. Preferred dosage forms are solutions, which contain a major amount of water in addition to the active ingredient. Minor amounts of other ingredients such as pH adjusters (e.g. a base such as NaOH), emulsifiers or dispersing agents, buffering agents, preservatives, wetting agents and jelling agents (e.g. methylcellulose) may also be present. Most preferably, the ophthalmic composition is a sterile, isotonic, buffered aqueous solution. Generally speaking, the ophthalmic composition containing the CAI may be prepared and may contain the various inert ingredients or carriers as previously described in the patent or non-patent literature as being suitable for ophthalmic compositions comprising carbonic anhydrase inhibitors. The amount of the CAI, which will be present in the ophthalmic composition will of course vary with the particular CAI employed and the type of formulation selected. Generally speaking, the composition will contain 0.01 to 5% of the CAI, preferably 0.25 to 2.5%; in other words, each mL of solution will contain 0.1 to 50 mg, preferably 2.5 to 25 mg, of the free base. The dose administered ophthalmically will be selected according to the particular compound employed, the size and condition of the patient and the effect desired, but in any event will be a quantity sufficient to prevent retinopathy.

Generally speaking, the carbonic anhydrase inhibitors will be used in the treatment of retinopathies in accord with this invention in dosages similar to those used for the treatment of glaucoma. The following are given by way of example: Dorzolamide (typically employed as the hydrochloride salt) can be administered ophthalmically as eye drops in 1-8% concentration, one to four times per day; it can also be administered orally or parenterally. Acetazolamide can be administered orally as tablets or capsules of 100 to 1000 mg per day or ophthalmically as eye drops in 0.5-5% (for example, 0.5-2%) concentration, one to four times per day. Methazolamide can be orally administered as tablets or capsules at a dosage level of 10-500 mg per day or ophthalmically as 0.5-5% eye drops, one to four times per day. Ethoxyzolamide can be orally administered as tablets or capsules at a dosage level of 10-500 mg per day or as 0.5-5% eye drops one to four times per day. Brinzolamide can be administered topically/ophthalmically as 1-5% eye drops, one to four times per day; it may also be administered orally or parenterally, of course.

### CLINICAL STUDY - PROGRESSION OF RETINOPATHY

In a retrospective clinical study, the hypothesis that dorzolamide eye drops (Trusopt®) used bid or tid for glaucoma or ocular hypertension would slow the progression or on set of diabetic retinopathy in patients with diabetes mellitus was tested.

### Methods

Since 1980, a screening program has been in place for diabetic eye disease at the Department of Ophthalmology, University of Iceland, Reykjavik, Iceland. Eye examinations of diabetic patients have been performed annually and examination results have been documented. A retrospective study was undertaken to find those diabetics who were using CAIs or beta-blockers (such as timolol) for either glaucoma or ocular hypertension. These candidates were selected from the general population of the screening program. In total, 31 patients were found. Of these 31, 19 were prescribed CAIs or a combination of CAIs and beta-blockers. The remaining 12 patients were taking a non-CAI medication and served as a control group in the study. The examination findings were reviewed to determine if the changes in retinopathy rating could be associated with CAI use. Also noted was historical information such as length of time since diabetes had been diagnosed, type of medication and time span of usage, visual acuity and other relevant comments.

### Patient Characteristics

Of those using a CAI, 12 were men and seven were women. The mean age was 67 (range 28-80) for men and 73 (range 62-81) for women. The average time since diagnosis of diabetes mellitus was 14.1 years (SD = 9.7 years). The average frequency of check-ups was every 9.1 months (SD = 10.7 months). There was an average of 7.4 years of eye examinations and fundus photographs on record (SD = 6 years) and the patients were on a CAI for an average of 18.2 months (SD = 14.4 months).

Of those in the non-CAI (control) group, seven were men and five were women. The mean age was 73 (range 54-82) for men and 75 (range 67-86) for women. The average time since diabetic diagnosis was 15.3 years (SD = 7.8 years). The average frequency of check-ups was every 10.9 months (SD = 7.9 months). There was an average of 6.2 years of eye examinations and fundus photographs on record (SD = 4 years) and the patients were on medication for an average of 58.8 months (SD = 52.4 months).

### Results

### CAI Group

Most patients were rated in stage 1 (no retinopathy) and stage 2 (background, nonproliferative retinopathy) on the retinopathy scale for the duration of their records. Stage 1 is no retinopathy and stage 2 is mild background (nonproliferative retinopathy). Over a period of 2 years, no individual had progression of retinopathy overall. The two year event rate was 0%. One individual experienced a progression from stage 1 to stage 2 after taking dorzolamide eye drops (Trusopt®) for one year. However, a year later, while on Trusopt® eye drops, the retinopathy regressed back to stage 1 (or no retinopathy).

### Non-CAI Group

There was more retinopathy change in this group. Three patients had retinopathies which progressed to stage 3 (diabetic macular edema), stage 5 (proliferative diabetic retinopathy), or stage 6 (advanced diabetic retinopathy), respectively, but only in one eye. Like the CAI group, however, retinopathy was mostly in stage 1 (no retinopathy) and stage 2 (background retinopathy) for the duration of the records. Four individuals experienced some type of change within this sample, which is a 25% event rate. Three of these had progressive changes occurring at 44, 45 and 59 months following the onset of glaucoma medication (timolol eye drops), respectively. The two year event rate counting from the onset of glaucoma medication thus was 8.3% (1 out of 12). This individual progressed from stage 1 to stage 2 after 19 months on glaucoma medication. The overall event rate recorded in this group was 25% for the entire observation period.

### Discussion

Those diabetics who were using dorzolamide eye drops on a regular basis appear to have a lower rate of progression of diabetic retinopathy than those diabetics who were not using dorzolamide and were receiving timolol eye drops for control of glaucoma or ocular hypertension. The rate of progression of diabetic retinopathy in the dorzolamide group was also lower than that found in this population of diabetics in a previous study in Iceland, where the yearly event rate in the progression of diabetic retinopathy exceeded 10% (Kristinsson et al., *Acta Ophthalmologica Scandinavica,* **75,** 249-254, 1997). The rate of progression of retinopathy is lower in the dorzolamide treated group than in either the total population of diabetics in Iceland or those diabetics treated with other glaucoma drugs, primarily beta-blockers.

Also, none of the diabetics in the dorzolamide treated group developed sight-threatening retinopathy during the treatment period. Indeed, no diabetic in Iceland has developed sight-threatening retinopathy while being treated with dorzolamide since the introduction of Trusopt® to the market.

The present conclusion that dorzolamide and other CAIs are preventative and/or therapeutic for diabetic retinopathy is based on a pathophysiologic rationale and the clinical experience recorded above.

### Diabetics with no retinopathy

Capillary occlusions are a hallmark of diabetic retinopathy and generally thought to be the underlying cause of further pathophysiological changes in the retina through hypoxia. Preventing the capillary occlusions prevents the development of diabetic retinopathy. Attempts are underway to reach this goal through agents that reduce clogging by leucocytes. The present invention proposes to prevent capillary occlusion through vasodilatation by CAIs.

## Claims

1. Use of a carbonic anhydrase inhibitor in the preparation of a medicament for the prevention of diabetic retinopathy in a diabetic not suffering from diabetic retinopathy.

2. Use according to Claim 1, wherein the medicament is prepared in a form suitable for ophthalmic administration to the eye or eyes.

3. Use according to Claim 1, wherein the medicament is prepared in a form suitable for systemic administration.

4. Use according to Claim 3, wherein the medicament is prepared in a form suitable for oral administration.

5. Use according to Claim 3, wherein the medicament is prepared in a form suitable for parenteral administration.

6. Use according Claim 1, wherein the carbonic anhydrase inhibitor is dorzolamide.

7. Use according to Claim 1, wherein the carbonic anhydrase inhibitor is acetazolamide.

8. Use according to Claim 1, wherein the carbonic anhydrase inhibitor is brinzolamide.

9. Use according to Claim 1, wherein the carbonic anhydrase inhibitor is methazolamide.

10. Use according to Claim 1, wherein the carbonic anhydrase inhibitor is ethoxyzolamide.

11. Use according to Claim 1, wherein the carbonic anhydrase inhibitor is butazolamide.

12. Use according to Claim 1, wherein the carbonic anhydrase inhibitor is dichlorphenamide.

13. Use according to Claim 1, wherein the carbonic anhydrase inhibitor is flumethiazide.

14. Use according to Claim 2, wherein the carbonic anhydrase inhibitor is dorzolamide, acetazolamide, methazolimide, ethoxyzolamide or brinzolamide.

15. Use according to Claim 4, wherein the carbonic anhydrase inhibitor is dorzolamide, acetazolamide, methazolamide, ethoxyzolamide or brinzolamide.

16. Use according to Claim 5, wherein the carbonic anhydrase inhibitor is dorzolamide or brinzolamide.

## Patentansprüche

1. Verwendung eines Carboanhydrase-Inhibitor zur Herstellung eines Medikaments zur Prävention von diabetischer Retinopathie bei einem Diabetiker, der nicht an diabetischer Retinopathie leidet.

2. Verwendung nach Anspruch 1, wobei das Medikament in einer Form hergestellt ist, die für eine ophthalmische Verabreichung an das Auge oder die Augen geeignet ist.

3. Verwendung nach Anspruch 1, wobei das Medikament in einer Form hergestellt ist, die zur systemischen Verabreichung geeignet ist.

4. Verwendung nach Anspruch 3, wobei das Medikament in einer Form hergestellt ist, die für eine orale Verabreichung geeignet ist.

5. Verwendung nach Anspruch 3, wobei das Medikament in einer Form hergestellt ist, die für eine parenterale Verabreichung geeignet ist.

6. Verwendung nach Anspruch 1, wobei der Carboanhydrase-Inhibitor Dorzolamid ist.

7. Verwendung nach Anspruch 1, wobei der Carboanhydrase-Inhibitor Acetazolamid ist.

8. Verwendung nach Anspruch 1, wobei der Carboanhydrase-Inhibitor Brinzolamid ist.

9. Verwendung nach Anspruch 1, wobei der Carboanhydrase-Inhibitor Methazolamid ist.

10. Verwendung nach Anspruch 1, wobei der Carboanhydrase-Inhibitor Ethoxyzolamid ist.

11. Verwendung nach Anspruch 1, wobei der Carboanhydrase-Inhibitor Butazolamid ist

12. Verwendung nach Anspruch 1, wobei der Carboanhydrase-Inhibitor Dichlorphenamid ist.

13. Verwendung nach Anspruch 1, wobei der Carboanhydrase-Inhibitor Flumethiazid ist.

14. Verwendung nach Anspruch 2, wobei der Carboanhydrase-Inhibitor Dorzolamid, Acetazolamid, Methazolimid, Ethoxyzolamid oder Brinzolamid ist.

15. Verwendung nach Anspruch 4, wobei der Carboanhydrase-Inhibitor Dorzolamid, Acetazolamid, Methazolamid, Ethoxyzolamid oder Brinzolamid ist.

16. Verwendung nach Anspruch 5, wobei der Carboanhydrase-Inhibitor Dorzolamid oder Brinzolamid ist.

## Revendications

1. Utilisation d'un inhibiteur de l'anhydrase carbonique pour la préparation d'un médicament destiné à la prévention de la rétinopathie diabétique chez un diabétique ne souffrant pas de rétinopathie diabétique.

2. Utilisation selon la revendication 1, dans laquelle le médicament est préparé sous une forme adaptée à une administration ophtalmique à l'oeil ou aux yeux.

3. Utilisation selon la revendication 1, dans laquelle le médicament est préparé sous une forme adaptée à une administration systémique.

4. Utilisation selon la revendication 3, dans laquelle le médicament est préparé sous une forme adaptée à une administration orale.

5. Utilisation selon la revendication 3, dans laquelle le médicament est préparé sous une forme adaptée à une administration parentérale.

6. Utilisation selon la revendication 1, dans laquelle l'inhibiteur de l'anhydrase carbonique est le dorzolamide.

7. Utilisation selon la revendication 1, dans laquelle l'inhibiteur de l'anhydrase carbonique est l'acétazolamide.

8. Utilisation selon la revendication 1, dans laquelle l'inhibiteur de l'anhydrase carbonique est le brinzolamide.

9. Utilisation selon la revendication 1, dans laquelle l'inhibiteur de l'anhydrase carbonique est le méthazolamide.

10. Utilisation selon la revendication 1, dans laquelle l'inhibiteur de l'anhydrase carbonique est l'éthoxyzolamide.

11. Utilisation selon la revendication 1, dans laquelle l'inhibiteur de l'anhydrase carbonique est le butazolamide.

12. Utilisation selon la revendication 1, dans laquelle l'inhibiteur de l'anhydrase carbonique est le dichlorphénamide.

13. Utilisation selon la revendication 1, dans laquelle l'inhibiteur de l'anhydrase carbonique est le fluméthiazide.

14. Utilisation selon la revendication 2, dans laquelle l'inhibiteur de l'anhydrase carbonique est le dorzolamide, l'acétazolamide, le méthazolamide, l'éthoxyzolamide ou le brinzolamide.

15. Utilisation selon la revendication 4, dans laquelle l'inhibiteur de l'anhydrase carbonique est le dorzolamide, l'acétazolamide, le méthazolamide, l'éthoxyzolamide ou le brinzolamide.

16. Utilisation selon la revendication 5, dans laquelle l'inhibiteur de l'anhydrase carbonique est le dorzolamide ou le brinzolamide.
